(19) Europäisches Patentamt
European Patent Office
Office européen des brevets

(11) Numéro de publication : **0 237 446**
**B1**

(12) # FASCICULE DE BREVET EUROPÉEN

(45) Date de publication du fascicule du brevet : **20.09.89**

(51) Int. Cl.[4] : **C 07 C145/00**, C 07 C143/08

(21) Numéro de dépôt : **87420020.7**

(22) Date de dépôt : **21.01.87**

(54) **Procédé de préparation d'acides perhalogénométhanesulfiniques et sulfoniques et de leurs sels.**

(30) Priorité : **06.02.86 FR 8601800**

(43) Date de publication de la demande : **16.09.87 Bulletin 87/38**

(45) Mention de la délivrance du brevet : **20.09.89 Bulletin 89/38**

(84) Etats contractants désignés : **AT BE CH DE ES FR GB GR IT LI LU NL SE**

(56) Documents cités : **EP-A- 0 165 135**

(73) Titulaire : **RHONE-POULENC CHIMIE**
**25, quai Paul Doumer**
**F-92408 Courbevoie Cédex (FR)**

(72) Inventeur : **Tordeux, Marc**
**1, rue Seignelay**
**F-92330 Sceaux (FR)**
Inventeur : **Langlois, Bernard**
**91, rue Duguesclin**
**F-69006 Lyon (FR)**
Inventeur : **Wakselman, Claude**
**5, rue Chaneze**
**F-75016 Paris (FR)**

(74) Mandataire : **Le Pennec, Magali et al**
**RHONE-POULENC INTERSERVICES Service Brevets Chimie 25, Quai Paul Doumer**
**F-92408 Courbevoie Cédex (FR)**

**EP 0 237 446 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

La présente invention concerne un procédé de préparation d'acides perhalogénométhanesulfiniques et sulfoniques et de leurs sels. Elle concerne plus particulièrement la préparation de sels d'acides difluorochloro, difluorobromo ou trifluorométhane sulfiniques et sulfoniques.

Ces acides perhalogénométhanesulfoniques et plus particulièrement l'acide trifluorométhanesulfonique sont utilisés comme catalyseurs ou comme intermédiaires en synthèse organique.

Les seuls procédés connus actuellement de fabrication de l'acide trifluorométhanesulfonique sont : la fluoration électrochimique telle que décrite notamment par R.D. Howells, J.D. Mc Cown dans Chemical reviews, 1977, 77, 69 ou le procédé de préparation décrit dans le brevet européen publié sous le numéro EP 165 135 qui consiste à mettre en présence du dioxyde de soufre, un métal choisi parmi le zinc, l'aluminium, le manganèse, le cadmium, le magnésium, l'étain, le fer, le nickel et le cobalt dans un solvant aprotique polaire puis à ajouter l'halogénure de trifluorométhyle sous une pression supérieure à 1 bar. Ce dernier procédé permet d'obtenir le trifluorométhane sulfinate avec de bons rendements. Le sulfinate obtenu se trouve dans un milieu contenant une quantité importante de sels de zinc. La séparation du sulfinate des autres sels de zinc pose au niveau industriel un problème difficile à résoudre.

Le procédé de préparation par voie électrochimique étant exclu de par son prix de revient industriel trop élevé, l'industrie est toujours à la recherche d'un procédé facile à mettre en œuvre et économiquement valable.

La présente invention a permis d'atteindre cet objectif et a pour objet un procédé de préparation de sels d'acides perhalogénométhanesulfiniques de formule générale (I)

$$[X_1X_2\,FC - SO_2]_{3-n}\,M \qquad (I)$$

dans laquelle $X_1$ et $X_2$ représentent un halogène identique ou différent choisi parmi F, Cl, Br, M représente un métal alcalin ou alcalino-terreux, n est égal à 1 ou 2 suivant l'état valentiel du métal M, caractérisé en ce qu'on met en présence dans un solvant aprotique polaire un dithionite alcalin ou alcalino-terreux de formule générale $M_n$ $(S_2O_4)$ (II), dans laquelle n a la même signification que précédemment, ou un hydroxyméthanesulfinate métallique ou alcalin et un perhalogénométhane.

Le procédé de la présente invention permet, contrairement à l'art antérieur précédemment cité et en particulier au brevet EP 165 135 de préparer les acides de la présente invention ou leurs sels en l'absence de catalyseur métallique.

Parmi les composés de formule (I) on peut citer les sels de sodium, potassium de l'acide trifluorométhanesulfinique, de l'acide chlorodifluorométhanesulfinique, de l'acide bromodifluorométhanesulfinique, de l'acide dichlorofluorométhanesulfinique. Le procédé de la présente invention est particulièrement adapté à la préparation des sels de l'acide trifluorométhanesulfinique.

Parmi les composés de formule (II), on préfère utiliser le dithionite de sodium ou de potassium. On préfère tout particulièrement utiliser le dithionite de sodium. Parmi les hydroxyméthanesulfinates, on préfère utiliser l'hydroxyméthanesulfinate de sodium plus connu sous le nom commercial de Rongalite ou l'hydroxyméthanesulfinate de zinc plus connu sous le nom commercial de Decroline ou encore l'hydroxyméthanesulfinate de cuivre. Peuvent être utilisés dans le cadre de la présente invention tous composés équivalents présentant la même fonction que les hydroxyméthanesulfinates c'est-à-dire tous les composés capables de libérer un radical anion sulfinique ($SO_2^{-}$) dans le milieu réactionnel.

Parmi les solvants polaires on préfère utiliser les amides tels que notamment : le formamide, le diméthylformamide, la N-méthylpyrrolidone, le diméthylacétamide et des solvants tels que le sulfolane. On préfère tout particulièrement utiliser le diméthylformamide.

Lorsque l'on utilise un dithionite de formule générale (II) ou un hydroxyméthanesulfinate on ajoute avantageusement une base choisie parmi les hydroxydes alcalins, alcalino-terreux, l'ammoniaque, la trisdioxa-heptylamine, le triéthylbenzylammonium, les sels d'acides faibles tels que par exemple le phosphate disodique, le métabisulfite de sodium, l'hydrogénosulfite de sodium, le borate de sodium. On préfère utiliser le phosphate disodique.

Parmi les perhalogénométhanes on préfère utiliser les perhalogénométhanes gazeux et parmi eux notamment le bromotrifluoro méthane, le bromochlorodifluorométhane, le dichlorodifluorométhane ou le trichlorofluorométhane ou le dibromodifluorométhane. Dans la classe des perhalogénométhanes gazeux, on préfère tout particulièrement utiliser le bromotrifluorométhane, moins onéreux car il fait partie des produits industriels utilisés notamment dans la lutte contre l'incendie. En effet ce composé est réputé pour son inertie chimique et il est particulièrement surprenant qu'en présence d'agents libérateurs de radical anion sulfinique il réagisse pour former un acide perhalogénométhanesulfinique.

Selon un premier procédé de mise en œuvre de l'invention avec le dithionite alcalin, celui-ci est introduit dans le réacteur en solution saturée dans l'eau ou le formamide. Il est même possible d'introduire le dithionite sous forme solide. On préfère éliminer tout l'oxygène présent dans le réacteur puis éventuellement on introduit du dioxyde de soufre et on introduit le perhalogénométhane.

Selon un deuxième procédé de mise en œuvre de l'invention quand on utilise un sel d'hydroxyméthanesulfinate, on introduit ce dernier directement sous forme solide dans le solvant réactionnel. On élimine l'oxygène présent dans le réacteur, puis éventuellement on introduit du dioxyde de soufre et on introduit le perhalogénométhane.

A la fin de la réaction on élimine le ou les solvants réactionnels puis on purifie le sel de l'acide sulfinique obtenu par extraction à l'aide de solvants tels que l'acétate d'éthyle. On transforme ensuite par oxydation le sel de l'acide sulfinique en sel de l'acide sulfonique de manière connue par tout homme de l'art. On obtient alors l'acide par exemple par acidification du sel par l'acide sulfurique anhydre de manière elle aussi connue par tout homme de l'art.

En ce qui concerne les conditions de réactions il est préférable de travailler à une température comprise entre 20° et 85 °C et encore plus préférentiellement lorsque l'on utilise un dithionite à une température comprise entre 65° et 75 °C.

Lorsque l'on travaille avec un gaz peu soluble dans le solvant de réaction, la pression réactionnelle est généralement supérieure à 1 bar. Une pression comprise entre 1 et 50 bars est préférée bien que la limite supérieure ne soit pas un critère essentiel mais uniquement préférentiel du point de vue technique.

De préférence le réacteur ne doit pas être constitué d'un matériau réactif tel que ceux décrits dans la demande de brevet publiée sous le numéro EP 165 135. On préfère ainsi utiliser un réacteur en verre.

Les acides perhalogénosulfoniques et leurs sels obtenus par le procédé de la présente invention sont utilisés comme catalyseurs ou comme intermédiaires de synthèse dans l'industrie pharmaceutique ou phytosanitaire.

L'invention va être plus complètement décrite à l'aide des exemples suivants qui ne doivent pas être considérés comme limitatifs de l'invention.

## Exemple 1

Dans un autoclave en verre de 0,6 litre, on introduit 80 g de dithionite de sodium, 60 g d'hydrogènophosphate de sodium, 120 ml d'eau et 80 ml de diméthylformamide. On fait le vide dans l'autoclave puis on le thermostate à 65 °C. Le mélange est ensuite agité sous une pression de 8 atmosphères de bromotrifluorométhane pendant 2 heures. L'autoclave est ouvert.

Les solvants contenus dans la phase supérieure sont évaporés sous vide. On rajoute 200 ml d'eau distillée. Les produits insolubles sont éliminés par filtration ; l'eau est évaporée sous vide. Les cristaux sont extraits à l'acétate d'éthyle bouillant. Après évaporation les cristaux sont séchés à 60 °C sous 0,5 mm Hg. On ajoute 40 ml d'eau oxygénée. On agite pendant 5 heures, puis le mélange est porté à ébullition pendant 4 heures avec de la pierre ponce. Les matières insolubles sont filtrées. L'eau est évaporée sous vide et les cristaux obtenus sont extraits à l'acétone bouillante. Après évaporation sous vide et séchage à 60 °C sous 0,5 mm de Hg. On obtient 19 g de trifluorométhanesulfonate de sodium — Rdt 24 %.

## Exemples 2 à 17

Dans un flacon en verre épais, on introduit 15 ml d'eau, 10 ml d'un solvant organique 10 g de dithionite de sodium et une base. On fait le vide dans un flacon, puis du bromotrifluorométhane est introduit dans le flacon. Il est alors agité pendant le temps indiqué dans le tableau, et la pression en bromotrifluorométhane est maintenue entre 3,5 et 2,4 atmosphères. Le flacon est ouvert, on récupère par décantation la phase supérieure, on ajoute une quantité connue de trifluoroéthanol. Le rendement en trifluorométhylsulfinate de sodium [(δ F = — 87 ppm) CF $Cl_3$] est évalué en résonance magnétique nucléaire du 19 F par intégration par rapport au trifluoroéthanol (δ F = — 76 ppm). Les résultats sont indiqués dans le tableau page suivante.

(Voir Tableau page 4)

Tableau de l'exemple 2 à 17

| | Solvant | Base | T° C | Temps | Rendement | |
|---|---|---|---|---|---|---|
| 2 | DMF | 3g Na OH | 85° | 2 h | 10 % | |
| 3 | DMF | 10g Na OH | 85° | 2 h | 6 % | |
| 4 | DMF | $NH_4OH$ | 65° | 2 h | 19 % | l'eau est remplacée par l'ammoniaque pure. |
| 5 | DMF | 6g $Na_2$ $HPO_4$ | 85° | 1 h | 19 % | |
| 6 | DMF | 6g $Na_2$ $HPO_4$ | 65° | 2 h | 21 % | à 8 atmosphères en $CF_3$ Br - Rdt : 29 % |
| 7. | DMF | 6g $Na_2$ $HPO_4$ | 45° | 3 h | 9 % | |
| 8 | DMF | 6g $Na_2$ $HPO_4$ + 3 g Fe | 65° | 4 h | 23 % | réaction plus lente |
| 9 | DMF | 6 g Zn | 65° | 6 h | 18 % | réaction lente non terminée |
| 10 | DMF | $Na_2$ $B_4O_7$ | 85° | 1 h | 9 % | |
| 11 | DMF | 5g $Na_2$ $S_2$ $O_5$ | 65° | 2 h | 4 % | |
| 12 | Ac OEt | 6g $Na_2$ $HPO_4$ | 65° | 3 h | trace | |
| 13 | pyridi-ne | 6g $Na_2$ $HPO_4$ | 65° | 2 h | 2 % | |
| 14 | $HCONH_2$ | 6g $Na_2$ $HPO_4$ | 65° | 1 h | 5 % | sans eau, l'eau est remplacée par le formamide (25 ml de formamide) |
| 15 | DMF | 8g $Na_2$ $HPO_4$ | 75° | 2 h | 23 % | |
| 16 | DMF | 8g $Na_2$ $HPO_4$ | 55° | 2 h | 10 % | non terminé |
| 17 | DMF | 6g $Na_2$ $HPO_4$ | 65° | 2 h | 17 % | sans eau, l'eau est remplacée par 15 ml de formamide |

Exemple 18

Dans un flacon en verre épais, on introduit 15 ml d'eau, 10 ml de diméthylformamide, 10 g de dithionite de sodium et 3 g de sodium hydrogénophosphate. On fait le vide dans le flacon, puis du bromochlorodifluorométhane est introduit dans le flacon. Il est alors agité pendant deux heures et la pression en bromochlorodifluorométhane est maintenue entre 2,5 et 1,5 atmosphères. Le flacon est

ouvert on récupère par décantation la phase supérieure on ajoute une quantité connue de trifluoroéthanol. Le rendement en chlorodifluoro-méthyl sulfinate de sodium (δ F = — 69 ppm) est évalué en résonance magnétique nucléaire du 19 F par intégration par rapport au trifluoroéthanol — Rendement 13 %.

Exemple 19

Dans un flacon en verre épais, on introduit 15 ml d'eau, 10 ml de diméthylformamide, 10 g de dithionite de sodium et 8 g d'hydrogénophosphate de sodium. On fait le vide dans le flacon puis du dichlorodifluorométhane est introduit dans le flacon. Il est alors agité pendant 3 heures, et la pression en dichlorodifluorométhane est maintenue entre 3,5 et 2,4 atmosphères. Le flacon est ouvert, on récupère par décantation la phase supérieure. Le chloro difluorométhylsulfinate de sodium est identifié par résonance magnétique nucléaire du 19 F.

Exemple 20

Dans un flacon en verre épais, on place 50 ml de diméthylformamide et 12,7 g d'hydroxyméthanesulfinate de zinc. On y fait le vide puis on le thermostate à 65 °C. Le mélange est agité pendant 3 heures sous une pression de bromotrifluorométhane comprise entre 3 et 5 bars. Le flacon est ensuite ouvert. Les solides sont éliminés par filtration et le diméthylformamide distillé sous vide. On ajoute 100 ml d'eau. Après élimination des solides par filtration, on ajoute de l'hydroxyde de sodium jusqu'à ce que le pH devienne basique, puis le pH est ramené à 7 par adjonction d'acide chlorhydrique. Les solides sont éliminés par filtration. Les cristaux sont extraits à l'acétate d'éthyle. L'acétate d'éthyle est évaporé sous vide. On agite avec 15 ml d'eau oxygénée à 35 % pendant 12 heures, puis le mélange est porté au reflux en présence de pierre ponce jusqu'à ce qu'il n'y ait plus de dégagement gazeux. Les solides sont éliminés par filtration. L'eau est évaporée sous vide et les cristaux sont repris avec de l'acétone. Après évaporation on obtient 5,5 g de trifluorométhanesulfonate de sodium.

Exemples 21 à 34

Dans un réacteur de 400 ml on charge en solution 150 ml d'eau et 32,6 g de phosphate disodique (0,23 mole), puis on charge 46,5 g (0,24 mole) de dithionite de sodium (pureté : 86,5 %) et pour terminer 100 g de diméthylformamide.

Après fermeture du réacteur, on met sous vide (10 mm de Hg) puis on admet le trifluorobromométhane sous une pression de 13 bars.

On agite et chauffe jusqu'à 65 °C quand la pression redescend à 12 bars on réintroduit à nouveau $CF_3Br$.

La durée totale de l'essai est de 1 h 10 mn. Après refroidissement et dégazage, on décante. La couche supérieure est distillée à sec. Le solide obtenu est lavé au chlorure de méthylène puis à l'acétate d'éthyle, on récupère ainsi le triméthylsulfinate de sodium avec un rendement de 69 % par rapport au dithionite chargé.

Exemples de 21 à 34

| N°Ex | Réactifs | | | | Con. opératoires | | Résultats |
|---|---|---|---|---|---|---|---|
| | $S_2O_4Na_2$ mole | eau/DMF $cm^3$ | $Na_2HPO_4$ / $NaH_2PO_4$ mole | $CF_3Br$ bar | Durée totale | temp. en °C | Rdt. % $CF_3SO_2Na$ |
| 21 | 0,240 | 150/100 | 0,23/0 | 13 | 1h 10 | 65 | 69 |
| 22 | 0,230 | 170/ 60 | 0,23/0 | 13 | 3h | 45 | 17 |
| 23 | 0,230 | 170/ 60 | 0,23/0 | 13 | 1h 40 | 65 | 23 |
| 24 | 0,230 | 150/100 | 0,12/0,12 | 13 | 1h 30 | 65 | 29 |
| 25 | 0,225 | 150/100 | 0,20/0 | 8 | 1h 15 | 65 | 58 |
| 26 | 0,200 | 125/125 | 0,20/0 | 8 | 1h | 65 | 60 |
| 27 | 0,225 | 125/120 | 0,23/0 | 8 | 1h | 65 | 43 |

(Suite)

| N°Ex | Réactifs $S_2O_4Na_2$ mole | eau/DMF $cm^3$ | $Na_2HPO_4$ / $NaH_2PO_4$ mole | $CF_3Br$ bar | Con. opératoires Durée totale | temp. en °C | Résultats Rdt. % $CF_3SO_2Na$ |
|---|---|---|---|---|---|---|---|
| 28 | 0,225 | 125/125 | 0,23/0 | 11 | 1h | 65 | 67 |
| 29 | 0,230 | 150/100 | 0,23/0 | 10 | 3h | 55 | 59 |
| 30 | 0,225 | 150/100 | 0,12/0 | 13 | 1h | 65 | 64 |
| | $S_2O_4Na_2$ mole | eau/NMP $cm^3$ | $Na_2HPO_4$ / $NaH_2PO_4$ mole | $CF_3Br$ bar | Durée totale | temp. en °C | Rdt. % $CF_3SO_2Na$ |
| 31 | 0,230 | 150/100 | 0,23/0 | 13 | 1h | 65 | 35 |
| 32 | 0,205 | 150/100 | 0,23/0 | 13 | 1h | 65 | 51 |
| 33 | 0,215 | 200/ 80 | 0,18/0 | 13 | 1h 10 | 65 | 28 |
| 34 | 0,230 | 150/100 | 0,12/0,12 | 13 | 0h 35 | 65 | 31 |

Exemples 35 à 41

Préparation du trifluorométhanesulfinate de zinc en présence de Décroline(R)/$CF_3Br$

Dans un flacon en verre épais, on place 50 ml d'un solvant organique et 12,7 g d'hydroxyméthanesulfinate de zinc. On y fait le vide puis on le thermostate à la température indiquée dans le tableau. On introduit dans l'exemple 37,2 g de dioxyde de soufre. Le mélange est agité sous une pression de bromotrifluorométhane comprise entre 3 et 5 bars pendant 3 heures ou jusqu'à ce qu'il n'y ait plus d'absorption de gaz. Le flacon est ensuite ouvert. On ajoute une quantité connue de trifluoroéthanol. Le rendement en trifluorométhanesulfinate de zinc [$\delta$ F = — 86 ppm/$CFCl_3$] est évalué en résonance magnétique nucléaire du 19 F par rapport au trifluoroéthanol ($\delta$ F = — 76 ppm). Les résultats calculés par rapport à la décroline(R) sont indiqués dans le tableau.

| n°E | Solvant | T°C | $SO_2$ | Rdt |
|---|---|---|---|---|
| 35 | DMSO | 20° | - | 22 % |
| 36 | DMSO | 50° | - | 31 % |
| 37 | DMF | 20° | + | 30 % |
| 38 | DMF | 65° | - | 35 % |
| 39 | DMF | 70° | - | 29 % |
| 40 | DMF | 80° | - | 15 % |
| 41 | NMP | 65° | - | 5 % |

Exemple 42

Préparation du trifluorométhanesulfonate de sodium

Dans un flacon en verre épais, on place 50 ml de diméthylformamide et 12,7 g d'hydroxyméthanesulfinate de sodium. On fait le vide puis on le thermostate à 75 °C. Le mélange est agité pendant 3 heures sous une ession de bromotrifluorométhane comprise entre 3 et 5 bars. Le flacon est ensuite ouvert. Les solides sont éliminés par filtration et le diméthylformamide distillé sous vide. On ajoute 100 ml d'eau. Après élimination des solides par filtration, on ajoute de l'hydroxyde de sodium jusqu'à ce que le pH devienne basique, puis le pH est ramené à 7 par adjonction d'acide chlorhydrique. Les solides sont éliminés par filtration. Les cristaux sont extraits à l'acétate d'éthyle. L'acétate d'éthyle est évaporé sous vide. On agite avec 15 ml d'eau oxygénée à 35 % pendant 12 heures ; puis le mélange est porté au reflux en présence de pierre ponce jusqu'à ce qu'il n'y ait plus de dégagement gazeux. Les solides sont éliminés par filtration. L'eau est évaporée sous vide et les cristaux sont repris avec de l'acétone. Après évaporation on obtient 5 g de trifluorométhanesulfonate de sodium.

Exemples 43 à 55 :

Préparation du trifluométhanesulfinate de sodium en présence d'hydroxyméthanesulfinate de sodium/$CF_3Br$

Dans un flacon en verre épais, on place 50 ml d'un solvant organique et 16,8 g d'hydroxyméthanesulfinate de sodium. Dans certains cas, on introduit ensemble ou séparément 2 g d'eau, 2 g de dioxyde de soufre, un agent de transfert de phase ou une base. Le mélange est placé sous vide et thermostaté à la température indiquée dans le tableau. Le flacon est agité sous une pression de bromotrifluorométhane comprise entre 3 et 5 bars pendant 3 heures ou jusqu'à ce qu'il n'y ait plus d'absorption de gaz. Le flacon est ensuite ouvert. On ajoute une quantité connue de trifluoroéthanol. Les rendements en trifluorométhanesulfinate de sodium [δ F = — 86 ppm/$CFCl_3$] sont évalués en résonance magnétique nucléaire du 19 F par intégration par rapport au trifluoroéthanol (δ F = — 76 ppm). Ils sont calculés par rapport à l'hydroxyméthanesulfinate de sodium et sont indiqués dans le tableau ci-dessous.

| n°E | Solvant | T°C | $H_2O$ | Base | $SO_2$ | Rdt |
|---|---|---|---|---|---|---|
| 43 | DMF | 50° | - | - | - | 16 % |
| 44 | DMF | 20° | - | - | - | 13 % |
| 45 | DMF | 20° | - | - | + | 20 % |
| 46 | DMF | 20° | + | - | + | 18 % |
| 47 | DMF | 20° | - | 0,5g TDA1 | + | 15 % |
| 48 | DMF | 20° | + | - | - | 18 % |
| 49 | DMF | 20° | - | 1g TEBA | + | 15 % |
| 50 | DMF | 20° | + | - | 4g $SO_2$ | 22 % |
| 51 | DMF | 20° | - | 10g $Na_2HPO_4$ | + | 21 % |
| 52 | DMF | 20° | - | 10g $Na_2S_2O_5$ | + | 28 % |
| 53 | NMP | 20° | - | - | + | 15 % |
| 54 | NMP | 20° | + | - | + | 21 % |
| 55 | NMP | 20° | + | 0,5g TDA1 | + | 18 % |

TDAI = trisdioxaheptylamine — TEBA = triéthylbenzylammonium.

Exemple 56

Préparation du chlorodifluorométhanesulfochlorure en présence de Décroline(R)/$CF_2BrCl$

Dans un flacon en verre épais, on place 50 ml d'un solvant organique et 12,7 g d'hydroxyméthanesulfinate de zinc. On fait le vide dans le flacon puis on agite le mélange pendant 15 heures sous une pression

de 1,2 bar de bromochlorodifluorométhane. Le flacon est ensuite ouvert. Les solides sont éliminés par filtration. On ajoute une quantité connue de trifluoroéthanol. Le rendement en chlorodifluorométhanesulfinate [δ F = — 69 ppm/$CFCl_3$] est évalué en résonance magnétique nucléaire du 19 F par rapport au trifluoroéthanol (δ F = — 76 ppm). Rendement 20 %. On ajoute 30 ml d'eau. Le diméthylformamide est extrait au dichlorométhane. Après filtration, on fait passer 2 l de chlore à travers la solution aqueuse et on obtient 3,2 g de chlorodifluorométhanesulfochlorure (δ F = — 58 ppm). Le rendement par rapport à l'hydroxyméthanesulfonate de zinc est de 18 %.

Exemple 57

Préparation du chlorodifluorométhanesulfinate de sodium en présence d'hydroxyméthanesulfinate de sodium/$CF_2Cl_2$

Dans un flacon en verre épais, on place 50 ml de diméthylformamide, 16,2 g d'hydroxyméthanesulfinate de sodium et 2 g de dioxyde de soufre. On fait le vide dans le flacon puis on agite le mélange pendant 2 heures sous une pression de dichlorodifluorométhane comprise entre 3 et 5 bars. Le flacon est ensuite ouvert. Le chlorodifluorométhanesulfinate de sodium (δ F = — 69 ppm) est identifié par résonance magnétique nucléaire du 19 F.

Exemple 58

Préparation du dichlorofluorométhanesulfinate de sodium en présence d'hydroxyméthanesulfinate de sodium/$FCCl_3$

Dans un erlenmeyer on place 10 ml de diméthylformamide, 3,7 g d'hydroxyméthanesulfinate de sodium et 2,74 g de trichlorofluorométhane. Le mélange est agité pendant 12 h. Le taux de conversion en trichlorofluorométhanesulfinate de sodium (δ F = —66 ppm) est de 33 % par rapport au trichlorofluorométhane et de 17 % par rapport à l'hydroxyméthanesulfinate de sodium.

**Revendications**

1. Procédé de préparation de sels d'acides perhalogénométhanesulfiniques de formule générale (I)

$$[X_1X_2\,FC — SO_2]_{3-n}\,M \qquad (I)$$

dans laquelle $X_1$ et $X_2$ représentent un halogène identique ou différent choisi parmi F, Cl, Br, M représente un métal alcalin ou alcalino-terreux, n est égal à 1 ou 2 suivant l'état valentiel du métal M, caractérisé en ce qu' on met en présence dans un solvant polaire un dithionite alcalin ou alcalino-terreux de formule générale $M_n$ ($S_2O_4$) (II) dans laquelle n et M ont la signification précédente, ou un hydroxyméthanesulfinate et un perhalogénométhane.

2. Procédé selon la revendication 1, caractérisé en ce que le perhalogénométhane est choisi parmi le bromotrifluorométhane, le bromochlorodifluorométhane, le dibromodifluorométhane, le dichlorodifluorométhane, le trichlorofluorométhane.

3. Procédé selon la revendication 1, caractérisé en ce que le solvant est le diméthylformamide.

4. Procédé selon la revendication 1, caractérisé en ce que le dithionite alcalin est le dithionite de sodium.

5. Procédé selon la revendication 1, caractérisé en ce que l'hydroxyméthanesulfinate est choisi parmi l'hydroxyméthanesulfinate de sodium, de zinc ou de cuivre.

6. Procédé selon la revendication 1, caractérisé en ce que l'on ajoute une base choisie de préférence parmi les hydroxydes alcalins, alcalino-terreux, l'ammoniaque, les sels d'acides faibles.

7. Procédé selon la revendication 6, caractérisé en ce que les sels d'acides faibles sont choisis parmi le phosphate disodique, le métabisulfite de sodium, l'hydrogénosulfite de sodium, le borate de sodium, et de préférence on choisit le phosphate disodique.

8. Procédé selon la revendication 1, caractérisé en ce que la température réactionnelle est comprise entre 20 °C et 85 °C et de préférence entre 65° et 75 °C lorsque l'on utilise le dithionite.

9. Procédé selon la revendication 1, caractérisé en ce que la pression réactionnelle est comprise entre 1 et 50 bars.

10. Procédé de préparation de sels d'acides perhalogénométhanesulfoniques, caractérisé en ce que dans une première étape on prépare un sel d'acide sulfinique selon l'une quelconque des revendications 1 à 9 et en ce que dans une 2e étape, on oxyde le sel d'acide sulfinique en sel d'acide sulfonique.

**Claims**

1. A process for the preparation of perhalomethanesulphinic acid salts of general formula (I)

$$[X_1X_2\ FC — SO_2]_{3-n}\ M \quad (I)$$

in which $X_1$ and $X_2$ represent an identical or different halogen atom chosen from F, Cl and Br, M represents an alkali metal or alkaline-earth metal, n is equal to 1 or 2 depending on the valency state of the metal M, characterized in that an alkali metal or alkaline-earth metal dithionite of general formula $M_n$ $(S_2O_4)$ (II) in which n and M have the above meaning, or a hydroxymethanesulphinate is brought into contact with a perhalomethane in a polar solvent.

2. The process according to claim 1, characterized in that the perhalomethane is chosen from bromotrifluoromethane, bromochlorodifluoromethane, dibromodifluoromethane, dichlorodifluoromethane and trichlorofluoromethane.

3. The process according to claim 1, characterized in that the solvent is dimethylformamide.

4. The process according to claim 1, characterized in that the alkali metal dithionite is sodium dithionite.

5. The process according to claim 1, characterized in that the hydroxymethanesulphinate is chosen from sodium, zinc or copper hydroxymethanesulphinate.

6 The process according to claim 1, characterized in that a base preferably chosen from alkali metal or alkaline-earth metal hydroxides, ammonia and salts of weak acids is added.

7. The process according to claim 6, characterized in that the salts of weak acids are chosen from disodium phosphate, sodium metabisulphite, sodium hydrogen sulphite and sodium borate, and preferably disodium phosphate is chosen.

8. The process according to claim 1, characterized in that the reaction temperature is between 20 °C and 85 °C and preferably between 65° and 75 °C when the dithionite is used.

9. The process according to claim 1, characterized in that the reaction pressure is between 1 and 50 bars.

10. A process for the preparation of perhalomethanesulphonic acid salts, characterized in that in a first stage, a sulphinic acid salt is prepared according to any one of claims 1 to 9 and in that in a second stage, the sulphinic acid salt is oxidized into sulphonic acid salt.

**Patentansprüche**

1. Verfahren zur Herstellung von Salzen von Perhalogenmethansulfinsäuren der allgemeinen Formel (I)

$$[X_1X_2\ FC — SO_2]_{3-n}\ M \quad (I)$$

worin $X_1$ und $X_2$ ein gleiches oder verschiedenes Halogen, ausgewählt aus F, Cl, Br, darstellen, M ein Alkali- oder Erdalkalimetall darstellt, n 1 oder 2 ist, je nach Wertigkeit des Metalls M, dadurch gekennzeichnet, daß man in einem polaren Lösungsmittel ein Alkali- oder Erdalkalidithionit der allgemeinen Formel $M_n$ $(S_2O_4)$ (II), worin n und M die vorstehende Bedeutung haben, oder ein Hydroxymethansulfinat und ein Perhalogenmethan zusammenbringt.

2. Verfahren nach Anspruch 1, dadurch gekennzeichnet, daß das Perhalogenmethan aus Bromtrifluormethan, Bromchlordifluormethan, Dibromdifluormethan, Dichlordifluormethan, Trichlorfluormethan ausgewählt wird.

3. Verfahren nach Anspruch 1, dadurch gekennzeichnet, daß das Lösungsmittel Dimethylformamid ist.

4. Verfahren nach Anspruch 1, dadurch gekennzeichnet, daß das Alkalidithionit Natriumdithionit ist.

5. Verfahren nach Anspruch 1, dadurch gekennzeichnet, daß das Hydroxymethansulfinat aus Natrium-, Zink- oder Kupferhydroxymethansulfinat ausgewählt wird.

6. Verfahren nach Anspruch 1, dadurch gekennzeichnet, daß man eine vorzugsweise aus Alkali-, Erdalkali-, Ammoniumhydroxiden sowie den Salzen schwacher Saüren ausgewählte Base zufügt.

7. Verfahren nach Anspruch 6, dadurch gekennzeichnet, daß die Salze der schwachen Säuren aus Dinatriumphosphat, Natriummetabisulfit, Natriumhydrogensulfit, Natriumborat ausgewählt werden und man vorzugsweise Dinatriumphosphat wählt.

8. Verfahren nach Anspruch 1, dadurch gekennzeichnet, daß die Reaktionstemperatur zwischen 20 °C und 85 °C und vorzugsweise zwischen 65 und 75 °C, wenn man Dithionit verwendet, liegt.

9. Verfahren nach Anspruch 1, dadurch gekennzeichnet, daß der Reaktionsdruck zwischen 1 und 50 bar liegt.

10. Verfahren zur Herstellung von Salzen von Perhalogenmethansulfonsäuren, dadurch gekennzeichnet, daß man in einem ersten Schritt ein Sulfinsäuresalze nach einem der Ansprüche 1 bis 9 bildet und dann in einem zweiten Schritt das Sulfinsäuresalze zum Sulfonsäuresalz oxidiert.